(19) Europäisches Patentamt
European Patent Office
Office européen des brevets

(11) Publication number: **0 188 010**
**A1**

# (12) EUROPEAN PATENT APPLICATION

(21) Application number: 85200022.3

(22) Date of filing: 14.01.85

(51) Int. Cl.[4]: C 07 J 7/00
C 07 J 71/00, C 07 J 31/00

(43) Date of publication of application: 23.07.86 Bulletin 86/30

(84) Designated Contracting States: NL

(71) Applicant: Gist - Brocades N.V.
Wateringseweg 1 P.O. Box 1
NL-2600 MA Delft(NL)

(72) Inventor: Smid, Peter Max
Rembrandtlaan 12
NL-2665 XG Bleiswijk(NL)

(72) Inventor: van Zoest, Willem Johan
Koekoekslaan 11
NL-3121 XJ Schiedam(NL)

(72) Inventor: Marx, Arthur Friedrich
Florence Nightingalelaan 12
NL-2614 GH Delft(NL)

(74) Representative: Possel, Okko et al,
c/o GIST-BROCADES N.V. Patents and Trademarks
Department Martinus Nijhofflaan 2 P.O. Box 1
NL-2600 MA Delft(NL)

## (54) New process for the preparation of certain steroids, especially intermediates for the preparation of proligestone and related compounds, and new intermediates formed in this process.

(57) The invention relates to a new process for the preparation of 14-alpha-17-alpha-dihydroxy-17-beta-substituted steroids by reaction of 14-alpha-hydroxy-17-oxo-steroids with metal organic compounds. Furthermore, the invention relates to the 14-alpha-17-alpha-dihydroxy-17-beta-substituted steroids prepared by the new process and their use in the production of 14-alpha-17-alpha-methylenedioxypregnane derivatives.

EP 0 188 010 A1

New process for the preparation of certain steroids, especially intermediates for the preparation of proligestone and related compounds, and new intermediates formed in this process.

The invention relates to a new process for the preparation of 14-alpha-17-alpha-dihydroxy-17-beta-substituted steroids by reaction of 14-alpha-hydroxy-17-oxo-steroids with metal organic compounds.

More particularly the invention relates to a process for the preparation of 14-alpha-17-alpha-dihydroxy-steroids having a 17-beta-side-chain which can be converted into a 17-beta-acyl side-chain. The new reaction of the invention is especially suitable for the preparation of intermediates for the synthesis of proligestone (14-alpha-17-alpha-propylidene-dioxy-pregn-4-ene-3,20-dione) and related compounds.

Steroids occur widely in nature, both in animals and plants. Many steroids are hormones, such as estrogens and cortisone, which are produced by the body's endocrine system and which are of great importance in the regulation of numerous body processes, such as growth and metabolism.

Over the last 35 to 40 years, steroid therapy, that is the medical augmentation of steroid hormone insufficiences in the body, is one of the major chapters in the history of medical progress. Steroids are used now as among others antiinflammatory agents, antiallergic agents, anabolic agents, diuretica and contraceptives.

During a long time steroids for medical use were prepared starting with the isolation of steroids from natural sources followed by partial chemical degradation and one or more chemical reactions in order to get the desired substituent pattern. The oldest examples of steroids isolated from natural sources, and which are still important as starting material for the preparation of steroids for medical use, are cholesterol, lanosterol and cholic acids. Afterwards, hecogenin and diosgenin became important raw materials too.

At the present moment cheap starting materials for the synthesis of pharmaceutical active steroids are available by microbiological degradation of natural steroids. Especially a number of 17-oxo-steroids can be made by degradation of the abundant soybean derived sterols sitosterol and campesterol in this way. 17-Oxo-steroids which may be prepared in this way are for instance androst-4-ene-3,17-dione and androsta-1,4-diene-3,17-dione. These androstane derivatives can be converted into other steroid compounds in manners known in the art.

In British patent 1218282 14-alpha-17-alpha-methylenedioxypregnane derivatives of the general formula I are described.

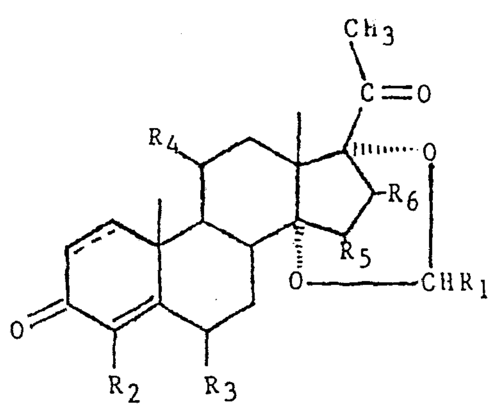

I

Compounds of the general formula I are therapeutically active compounds possesing progestational activity. Some of the compounds are particular useful in the treatment of threatened abortus, of dysmenorrhoea or for healing the mucous membrane of the uterus. Moreover, many of these compounds have excellent contraceptive properties. These compounds give in many cases a remarkably longlasting protection against conception. Therefore, they may be applied as longlasting contraceptives. Especially proligestone (formula II) is very suitable as a long-acting contraceptive due to its good anti-gonadotrophic activity, its weak progestogenic activity and the absence of any androgenic activity.

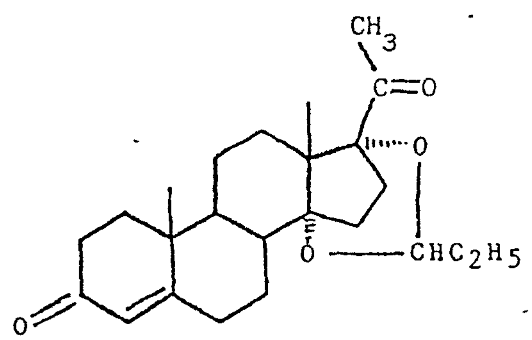

II

The pregnane derivatives of the general formula I are prepared by reacting a 14-alpha-17-alpha-dihydroxypregnane with an aldehyde of the general formula $R_1CHO$ or an acetal thereof of the general formula $R_1CH(OR_7)_2$ in which $R_7$ represents an alkyl group.

The 14-alpha-17-alpha-dihydroxypregnane derivatives which are used as starting materials in the processes hereinbefore described can be obtained by microbiological processes from the corresponding 17-alpha-hydroxy-pregnanes. For this process microorganisms that are capable of introducing a hydroxyl group at the 14-position are used. Various strains of the genera Curvularia, particularly Curvularia lunata, Helicostylum and Absidia regnieri, have been found to be very

suitable in this respect (see, for example, British Patent Specification No. 1,105,980).

The above mentioned 14-alpha-17-alpha-dihydroxypregnane derivatives can also be prepared from Reichstein's compound S, i.e. 17-alpha-21-dihydroxy-pregn-4-ene-3,20-dione, by microbiological conversion of this compound, e.g. with Curvularia lunata, into 14-alpha-17-alpha-21-trihydroxy-pregn-4-ene-3,20-dione, followed by reductive removal of the 21-hydroxy group (Tetrahedron Suppl. No. 7 (1966), 325). The 14-alpha-17-alpha-dihydroxy-pregn-4-ene-3,20-dione thus obtained may be converted into other pregnane derivatives by manners known per se.

It has appeared, however, that in practice the above described preparations of the dihydroxy starting materials are not entirely suitable. Also in view of the fact that cheap 17-oxo-steroids are available now on a large scale as discussed above, an extensive investigation was made to find a new syntheses for 14-alpha-17-alpha-dihydroxy-17-beta substituted steroids starting with 17-oxo-steroids.

As already described above 14-alpha-hydroxy groups may be introduced into the steroid molecule by microbiological processes. Microbiological 14-alpha-hydroxylation of 17-oxo-steroids, has been described, for example in Japanese patent application 6341/65, U.S. patent 3.243.355 and Dutch patent application 6715404.

It has now been found that 14-alpha-hydroxy-17-oxo-steroids, obtainable by microbiological 14-alpha-hydroxylation of 17-oxo-steroids, can easily be converted into 14-alpha-17-alpha-dihydroxy-17-beta-substituted steroids by reaction with certain metal organic compounds. The present invention, therefore, relates to a process for the preparation of 14-alpha-17-alpha-dihydroxy-17-beta-substituted steroids which comprises the reaction of a 14-alpha-hydroxy-17-oxo-steroid and a metal-organic compound in which the metal is lithium, sodium or potassium.

Nucleophilic addition at the 17-keto group of steroids are known. For instance, the reaction of 17-keto steroids with hydrogen cyanide (or acetone cyanohydrine) to form a mixture of 17-cyano-17-hydroxy compounds is well known.

Another example of nucleophilic addition is the reaction of a 17-keto steroid with a Grignard reagent to a 17-alkyl-17-hydroxy steroid. The stereoisomer formed in this reaction is predominantly the 17-alpha-alkyl-17-beta-hydroxy compound. For instance the orally effective androgen 17-alpha-methyltestosterone is prepared from 3-beta-hydroxyandrost-5-en-17-one by treatment with methylmagnesiumiodide to give the 17-alpha-methyl derivative, which is then oxidized. From large scale experiments the epimeric 3-beta-17-alpha-dihydroxy-17-beta-methyl-androst-5-ene could be isolated as a by-product. Reaction of 17-ketosteroids with benzylmagnesium chloride and allylmagnesium bromide resulted in the formation of 17-beta-hydroxy-17-alpha-substituted derivatives.

It may be concluded, therefore, that attack from the alpha-side of the molecule is preferred.

It has appeared that the steric course of the alkylation of 17-keto steroids is not influenced by an 11-keto group. However, the presence of a 12-alpha-OH group as well as a 16-alpha-acetoxy group renders alpha-side approach to the 17-oxo-group more difficult (Organic Reactions in Steroid Chemistry, Van Nostrand Reinhold Company, New York, Vol. 2, p.64). However, the reaction of 3-beta-acetoxy-14-alpha-hydroxy-androst-5-ene-17-one with allylmagnesiumbromide resulted in only one isomer, 3-beta-14-alpha-17-beta-trihydroxy-17-alpha-allylandrost-5-ene (Recl. trav. chim. Pays Bas, 82, 149 (1963). It may be concluded, therefore, that the presence of a 14-alpha-hydroxy group has little or no influence on the steric course of the reaction, and certainly does not make approach from the beta-side of the steroid more favorable.

In view of the above mentioned facts that attack from the alpha side of the molecule is preferred and that the presence of an 14-alpha-hydroxy group hardly influences the steric course of the reaction, it is to be expected that all

nucleophiles attacking the 17-keto-group would result in 17-beta-hydroxy-17-alpha substituted steroids when using 14-alpha-hydroxy steroids.

The process of the present invention, now, surprisingly resulted in the formation of the isomer at the 17-place of the steroid molecule wherein the nucleophile has come from the beta-side of the molecule, thus exclusively resulting in 14-alpha-17-alpha-dihydroxy-17-beta-substituted steroids. The presence of a 14-alpha-hydroxy group and a 17-alpha-hydroxy group makes the new process of the invention suitable for the preparation of the 14-alpha-17-alpha-methylenedioxypregnane derivatives described in British patent 1218282, more particularly when those nucleophiles are choosen which can be converted simply into an acetyl group.

The invention particularly relates to the reaction of 14-alpha-hydroxy-17-oxo-steroids with a metal organic compound, for instance an alkali metal acetylide and other organolithium, organosodium or organopotassium compounds.

Suitable metal-organic compounds are compounds with the formula R-M in which R represents a straight or branched alkyl, 1-alkenyl or 1-alkynyl group which optionally may contain a double bond and which optionally may be substituted by one or more substituents and M represents a lithium, sodium or potassium atom. Particularly R represents a straight or branched $C_{1-6}$ alkyl group, preferably methyl or ethyl, a $C_{1-6}$ 1-alkenylgroup, preferably ethenyl, or a $C_{1-6}$ 1-alkynylgroup, preferably ethynyl, which groups may be substituted by one or more $C_{1-4}$ alkoxy, aryloxy, tetrahydropyranyloxy, $C_{1-4}$ alkylthio, arylthio, $C_{1-4}$ alkylsulfinyl, arylsulfinyl, $C_{1-4}$ alkylsulfonyl, arylsulfonyl, $C_{1-4}$ dialkylamino, $C_{1-4}$ alkyl-arylamino, diarylamino, $C_{1-6}$ alkylhydrazono, $C_{1-4}$ alkyleneoxythio, $C_{1-4}$ alkylenethiosulfoxy or $C_{1-4}$ alkylenedithio groups, or a 1-(tri-$C_{1-4}$ alkylsilyloxy) or di $C_{1-6}$-dialkylamino group together with a 1-cyano group, the aryl groups preferably phenyl or naphtyl, optionally substituted by a methyl group. Preferably R represents an

ethyl group, an ethenyl group or an ethynyl group, which may be substituted by one or more $C_{1-4}$ alkoxy, phenyloxy, $C_{1-4}$ alkylthio, phenylthio, $C_{1-4}$ alkylsulfinyl, phenylsulfinyl, $C_{1-4}$ alkylsulfonyl, phenylsulfonyl, $C_{1-4}$ dialkylamino, $C_{1-4}$ alkyl-phenylamino, diphenylamino, $C_{1-4}$ alkyleneoxythio, $C_{1-4}$ alkylenethiosulfoxy or $C_{1-4}$ alkylenedithio group, the phenyl groups optionally substituted by a methyl group, or by a 1-(tri-$C_{1-4}$ alkylsilyloxy) group together with a 1-cyano group.

In particular those groups R are preferred which can be hydrolized into an acyl group, especially an acetyl group, and acetylides, which also can be converted into acyl groups. The first one of the groups is known in literature as the group of masked carbonyl groups, more particularly "umpolungs" reagents.

Preferred organometallic compounds are therefore those groups in which R represents an ethyl group substituted at the 1 position by two groups choosen from $C_{1-4}$ alkoxy, phenyloxy, $C_{1-4}$ alkylthio, phenylthio, $C_{1-4}$ alkylsulfinyl, phenylsulfinyl, $C_{1-4}$ alkylsulfonyl and phenylsulphonyl, or a 1-cyano group together with a tri-($C_{1-4}$ alkylsilyloxy)group or substituted at the 1 position with a $C_{1-4}$ alkyleneoxythio, $C_{1-4}$ alkylenethiosulfoxy or $C_{1-4}$ alkylenedithio groups, the phenyl groups optionally substituted with a methyl group, or R represents an ethenyl group substituted at the 1-position by a group choosen from $C_{1-4}$ alkoxy, phenyloxy, $C_{1-4}$ alkylthio, phenylthio, $C_{1-4}$ alkylsulfinyl, phenylsulfinyl, $C_{1-4}$ alkylsulfonyl and phenylsulphonyl, the phenyl group optionally substituted by a methyl group, or R represents an ethynyl group. It is remarked that in case R represents an acetylide group it is preferred to use lithium acetylide, especially in combination with ethylenediamine.

Suitable steroids for the process of the invention are 14-alpha-hydroxy-17-oxosteroids of the general formula III

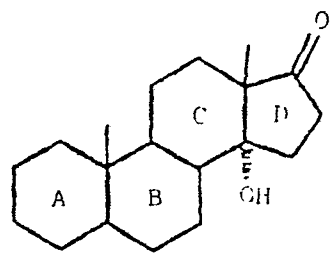

III

in which the rings A, B, C and D optionally contain one or more double bonds, are substituted or not substituted by one or more hydroxy groups, amino groups, oxygen atoms, or alkoxy or alkoxyalkoxy groups and are disubstituted or not disubstituted by one or more alkylenedioxy, alkylenedithio, alkylenethiosulfoxy or alkyloxythio groups.

When the rings A, B, C and D contain one or more double bonds, these double bonds are preferably present between $C_1$ and $C_2$, $C_3$ and $C_4$, $C_4$ and $C_5$, $C_5$ and $C_6$, $C_6$ and $C_7$, $C_9$ and $C_{11}$ and/or $C_{11}$ and $C_{12}$. More preferably the double bond is present between $C_4$ and $C_5$.

When two or more double bonds are present especially the following systems are preferred: $C_3$-$C_4$ and $C_5$-$C_6$, and $C_1$-$C_2$ and $C_4$-$C_5$.

When the rings A, B, C and D are substituted by a hydroxy group, suitable groups are 3-, 9-, 11- or 12-hydroxy groups.

When the rings A, B, C and D are substituted by an amino group, suitable amino groups are 3-alkylamino groups, preferably containing 1-4 carbon atoms, 3-dialkylamino groups wherein the alkyl groups are the same or different, each alkyl group preferably containing 1-4 carbon atoms, or amino groups in which the nitrogen atom together with the alkyl group form a heterocyclic ring, preferably containing 5-8 ring atoms,

which ring optionally may contain an oxygen atom. Particularly preferred are dimethylamino, diethylamino, pyrolidine and morfoline.

When the rings A, B, C and D are substituted by an oxygen atom suitable oxygen atoms are at $C_3$, $C_{11}$ or $C_{12}$, preferably at $C_3$.

When the rings A, B, C and D are substituted by an alkoxy group, suitable alkoxy groups are 3-, 9-, 11- or 12-alkoxy groups containing 1-4 carbon atoms, preferably 3-methoxy or ethoxy groups.

When the rings A, B, C and D are substituted by an alkoxyalkoxy group, suitable groups are 3-methoxymethoxy, methoxyethoxy or tetrahydropyranyloxy.

When the rings A, B, C and D are disubstituted, suitable substituents are a 3,3-alkylenedioxy, a 3,3-alkylenedithio, a 3,3-alkylene thiosulfoxy or a 3,3-alkyleneoxythio group. The alkylene group preferably contains 2 or 3 carbon atoms.

More particularly the invention relates to compounds which contain a keto group at $C_3$ and a double bonds between $C_1$ and $C_2$ and/or $C_4$ and $C_5$, or containing functional groups which can be converted into the keto group and double bond mentioned before.

Preferred 14-alpha-hydroxy-17-oxo-steroids are 14-alpha-hydroxy-androst-4-ene-3,17-dione and 14-alpha-hydroxy-androsta-1,4-diene-3,17-dione.

It is remarked that those steroids which contain one or more groups which would interfere during the reaction have to be protected at the relevant positions. This can be done by methods known in the art. In this respect it is remarked that a 3-oxo group or a 3-oxo-delta-4 group always have to be protected. This can be done, for instance, by converting the 3-oxo group in an enol ether or enamine derivative, or by conversion of the 3-oxo group in an alkylenedioxy, alkylenedithio or alkyleneoxythio group.

0188010

The reaction between the carbon nucleophile and the 14-alpha-hydroxy-17-oxo-steroid is carried out in an inert organic solvent medium. Suitable solvents are, for example, ethers such as tetrahydrofuran, diethyl ether, 1,2-dimethoxyethane, 1,2-diethoxyethane and dioxane, aromatic hydrocarbons such as benzene, toluene and xylene, aliphatic hydrocarbons such as pentane, hexane and cyclohexane, tetramethylethylenediamine and hexamethylphosphoric acid triamide and mixtures thereof. Preferred solvents are tetrahydrofuran and dioxane.

During the reaction the temperature is maintained between -80°C and 50°C, preferably maintained between -30° and 30°C.

It will be appreciated that another feature of the invention is formed by the new 14-alpha-17-alpha dihydroxy 17-beta-substituted compounds which can be prepared according to the process of the invention. Therefore, the invention also relates to 14-alpha-17-alpha-dihydroxy-17-beta-substituted steroids of the formula IV wherein R is as defined above and the substituents of the steroid molecule are also as defined above.

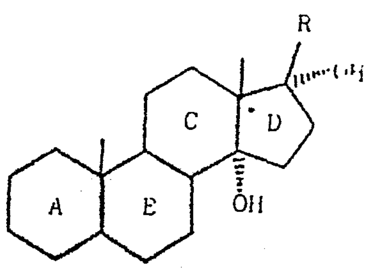

IV

Preferably those carbon nucleophiles are used having a group R which can be converted into an acyl group. Therefore R preferably represents an alkyl group, especially an ethyl group, substituted as described on page 7, a 1-alkenyl group, especially an ethenyl group, substituted as described on page 7, or an alkynyl group, especially an ethynyl group.

The conversion of the above described groups R into an acyl group may be performed by methods known in the art. As a general rule it may be said that the alkyl and 1-alkenyl group described in the paragraphs here-above may be hydrolized under aqueous acidic conditions. The conversion of an alkynyl group is generally performed using metal-ions, preferably mercury (II) compounds. For the conversion of the compounds of the general formula IV having a group R which may be converted into an acyl group, into compounds having the general formula V

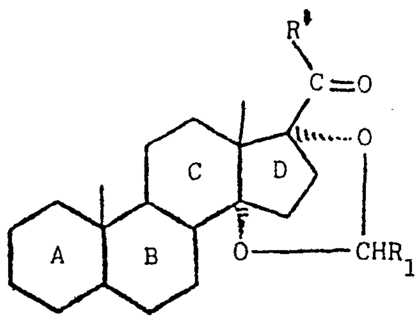

V

in which R' represents the remaining part of the group R after hydrolysis into the 17-beta-acyl group, $R_1$ represents a straight or branched chain aliphatic hydrocarbon group having less than 10 carbon atoms which is optionally substituted by a halogen atom or by a carboxyl or methoxycarbonyl group, an aryl group which is optionally substituted by a halogen atom, or by a $C_{1-6}$ alkyl, nitro or amino group or by one or more lower alkoxy groups or an aryl ($C_{1-6}$ alkyl) group, a $C_3$-$C_8$ cycloalkyl group, a lower alkoxy group or the furyl group, and the substituents of the steroid part of the molecule are as defined above, it is possible to use two different ways.

In the first way the 14-alpha-17-alpha-dihydroxy-17-beta-substituted steroid is reacted with an aldehyde of the general formula $R_1CHO$ or an acetal thereof or a suitable orthoformate, followed by conversion of the group R into an acyl group.

In the second way the group R is first converted into the acyl group, followed by reaction with the aldehyde of the general formula $R_1CHO$ or an acetal thereof or a suitable orthoformate.

In this respect it is also remarked that in case that one or more protective groups are present in the steroid skeleton due to the fact that otherwise one or more substituents would be present which would interfere in the reaction of the carbon nucleophile and the 17-oxo-group, these groups may be removed immediately after the reaction as well as after the conversion of the group R into an acyl group as also after the reaction with the aldehyde, or an acetal thereof or a suitable orthoformate. Sometimes it is possible to remove the protective groups simultaneously with the conversion of the group into the acyl group.

It will be appreciated that the use of the compounds of the general formula IV having a group R which may be converted into an acyl group for the preparation of compounds of the general formula V forms a feature of the invention. This helds especially for the use of these compounds for the preparation of proligestone, 14-alpha-17-alpha-propylidene-dioxy-pregna-4-ene-3,20-dione and related compounds. The invention, therefore, also relates to the preparation of compounds of the general formula V, starting with a compound of the general formula IV whereby R' represents the remaining part of the group R after hydrolysis into the 17-beta-acyl group, wherein R is defined as above, the substituents of the steroid molecule are as defined above and $R_1$ represents a straight or branched chain aliphatic hydrocarbon group having less than 10 carbon atom which is optionally substituted by a halogen atoms or by a carboxyl or methoxycarbonyl group, an aryl group which is

optionally substituted by a halogen atom, or by a $C_{1-6}$ alkyl, nitro or amino group or by one or more lower alkoxy groups or an aryl($C_{1-6}$ alkyl) group, a $C_3$-$C_8$ cycloalkyl group, a lower alkoxy group or the furyl group. For the preparation of proligestone 14-alpha-hydroxy-androst-4-ene-3,17-dione is used as the starting compound. The 3-oxo-delta-4-moiety of this molecule has to be protected, for instance by conversion into a compound of the general formula VI.

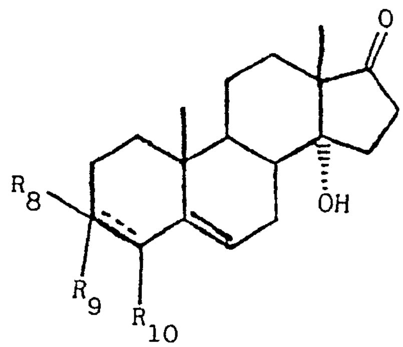

VI

wherein $R_8$ and $R_9$ are the same or different and each represents a group $-OR_{11}$ in which $R_{11}$ represents a $C_{1-6}$ alkyl group optionally substituted with a $C_{1-4}$ alkoxy group or $R_8$ and $R_9$ together represent an $C_{2-4}$ alkylenedioxy, alkylene-dithio, alkylenethiosulfoxy or alkyleneoxythio group and $R_{10}$ represents hydrogen, or $R_8$ represents a group $OR_{11}$ or a group

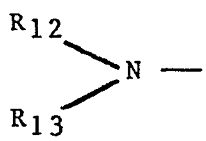 .

wherein $R_{12}$ and $R_{13}$ are the same or different and each represent an alkyl group, or $R_{12}$ and $R_{13}$ together with the nitrogen atom to which they are attached represent a 5- or 6-membered heterocyclic group, which may contain a further hetero atom in the ring and $R_9$ and $R_{10}$ together represent an additional bond in the 3-4 position. Preferably $R_8$ represents a group $OR_{11}$ in which $R_{11}$ represents a $C_{1-6}$ alkyl group, especially methyl or ethyl, and $R_9$ and $R_{10}$ together represent an additional double bond.

For the preparation of proligestone it is preferred to use for the preparation of the intermediate compounds of the general formula IV a metal organic compound choosen from the group lithium, sodium or potassium acetylide, especially lithium acetylide which is preferably used in combination with a complexing agents, for instance diaminoethane, 1-alkalimetal 1,1-trimethylenedithio-ethane, preferably the lithium derivative, 1-alkalimetal-1-$C_{1-6}$-alkylthio-ethene, preferably the lithium derivative and the methyl or ethyl group, 1-alkalimetal-1-phenylthio-ethene, preferably the lithium derivatives, the phenyl group optionally substituted with a methyl group, 1-alkalimetal-1-phenylsulfinyl ethene, preferably the lithium derivative, the phenyl group optionally substituted with a methyl group, 1-alkalimetal-1-phenylsulfonyl-ethene, preferably the lithium derivative, the phenyl group optionally substituted with a methyl group, 1-alkalimetal-1-$C_{1-6}$ alkoxy-ethene, preferably the lithium derivative and the methyl or ethyl group and 1-alkalimetal-1-phenoxy-ethene, preferably the lithium derivative, the phenyl group optionally substituted with a methyl group.

The compound of the general formula IV as described in the preceeding paragraph may be converted into proligestone by hydrolysis of the group R into an acetylgroup, followed by reaction with propionaldehyde, or by reaction with propionaldehyde followed by hydrolysis of the group R into an acetyl group. The conversion of the protected 3-oxo-delta-4 group into the 3-oxo-delta-4 may be performed directly after the reaction with the metal organic compound, or after the reaction with propionaldehyde, or after the hydrolysis of the group R. In some case the hydrolysis of the group R also results in the removal of the protective group.

The following Examples illustrate the present invention.

## Example I

**14-alpha-Hydroxy-3-methoxy-androsta-3,5-dien-17-one**

14-alpha-Hydroxy-androst-4-ene-3,17-dione (90 g, 0.30 mol) was suspended in a mixture of trimethylorthoformate (90 ml, 0.82 mol) and methanol (90 ml, 2.2 mol). Sulphuric acid in methanol (5% v/v, 0.5 ml) was added under vigorous stirring. After stirring for 2 hours at 25°C triethylamine (0.5 ml) was added. The solid mass was filtered off, washed with methanol and dried. Yield: 88.8 g (94%) of the title compound. NMR ($CDCl_3$, DMSO $d_6$) delta 0.975 (6H, s), 2.88 (1H, s), 3.55 (3H, s), 5.15 (1H, s), 5.26 (1H, m).

## Example II

**3-Ethoxy-14-alpha-hydroxy-androsta-3,5-dien-17-one**

Example I was repeated, however, triethylorthoformate was used instead of trimethylorthoformate. Yield 93%. NMR ($CDCl_3$, DMSO $d_6$) delta 1.003 (6H, s), 1.28 (3H, tr), 3.11 (1H,s) 3.73 (2H, q), 5.10 (1H, s), 5.21 (1H, m).

## Example III

**14-alpha-17-alpha-Dihydroxy-3-ethoxy-17-beta-ethynyl-androsta-3,5-diene**

3-Ethoxy-14-alpha-hydroxy-androsta-3,5-dien-17-one (30 g, 90.9 mmol) and lithium acetylide.ethylenediamine complex (30 g, 320 mmol) in dry dioxane (180 ml) were stirred for 20 hours at 25°C under a nitrogen atmosphere. After careful addition of water at 0°C (250 ml) the reaction mixture was stirred for half an hour during which the title compound crystallized. The solid mass was filtered, washed with water/dioxane (50/50), acetic acid (10% in water) and water, and dried. NMR ($CDCl_3$) delta 1.002 (3H, s), 1.059 (3H, s), 1.31 (3H, tr), 2.48 (1H, s), 3.12 (1H, s), 4.18 (1H, s), 3.80 (2H, q), 5.15 (1H, m), 5.25 (1H, s).

Example IV

14-alpha-17-alpha-Dihydroxy-17-beta-ethynyl-androst-4-en-3-one

The reaction product of Example III was suspended in a mixture of acetic acid (150 ml) and water (50 ml). Sulphuric acid (30%) was added to a pH of 0.5 was reached. After stirring for 1 hour at 25°C a solution of sodium hydroxide was added to neutralize the acid. Water was added (250 ml). The crystallized compound was filtered off, washed with acetic acid/water (50%) and dried. Yield: 28 g (92% calculated on 3-ethoxy-14-alpha-hydroxy-androsta-3,5-dien-17-one) of the title compound. NMR ($CDCl_3$) delta 1.051 (3H, s), 1.206 (3H, 1s), 2.51 (1H, s), 3.84 (1H, s), 4.45 (1H, s), 5.73 (1H, s).

Example V

17-beta-Ethynyl-14-alpha-17-alpha-propylidenedioxy-androst-4-en-3-one

The reaction product of Example IV was reacted with propanal (18 ml) and sulphuric acid (30% in acetic acid, 1 ml) in acetic acid (50 ml) at 25°C for 1 hour. After addition of water (75 ml) and sodium hydroxide solution (3 ml, 15% in water) the crystallized mass was filtered off and recrystallized from methanol (200 ml). Yield: 25.2 g (90%) of the title compound. NMR ($CDCl_3$) delta 0.94 (3H, tr), 1.023 (3H, s), 1.195 (3H, s), 2.51 (1H, s), 4.88 (1H, tr), 5.77 (1H, s).

Example VI

14-alpha-17-alpha-Propylidenedioxy-pregn-4-ene-3,20-dione

The reaction product of Example V (5 g, 13.6 mmol) was suspended in acetic acid (30 ml), water (6 ml), and a solution of sulphuric acid in acetic acid (0.75 ml, 30% v/v). Mercury (II) oxyde (300 mg, 1.38 mmol) was added. The reaction mixture was stirred for 2 hours at 50°C and cooled to 0°C, after which sodium hydroxide solution (2 ml, 15%) and water

were added. The crystallized mass was filtered off, washed with acetic acid in water (25%), dried and recrystallized from methanol. Yield: 3.84 g (73%) of the title compound. NMR ($CDCl_3$) delta 0.843 (3H, s), 0.96 (3H, tr), 1.178 (3H, s), 2.14 (3H, s), 4.86 (1H, tr), 5.68 (1H, s).

Example VII

14-alpha-17-alpha-Dihydroxy-pregn-4-ene-3,20-dione

14-alpha-17-alpha-Dihydroxy-17-beta-ethynyl-androst-4-en-3-one (2 g, 6.1 mmol) was suspended in a mixture of acetic acid (20 ml), water (4 ml) and a solution of sulphuric acid in acetic acid (0.5 ml, 30% v/v). Mercury (II) oxide (240 mg, 1.11 mmol) was added, and the reaction mixture was stirred for 2 hours at 30°C. Sodium hydroxide solution (0.5 ml, 15%) was added, followed by 10 ml of water. The crystallized mass was filtered off, washed and dried. Yield: 1.44 g of the title compound (68%). NMR ($CDCl_3$, $CD_3OD$) delta 0.77 (3H, s), 1.25 (3H, s), 2.22 (3H, s), 4.32 (2H, s), 5.73 (1H, s). The compound could be converted into 14-alpha-17-alpha-propylidenedioxy-pregn-4-ene-3,20-dione according to known methods.

Example VIII

14-alpha-17-alpha-Dihydroxy-3-ethoxy-17-beta-1-(1-phenylthio-ethenyl)-androsta-3,5-diene

To a solution of phenylthioethene (20 ml) and diisopropylamine (2 ml) in dry THF (200 ml) under a nitrogen atmosphere n-butyllithium in hexane (100 ml, 15%) was added in 30 minutes at -10°C. After stirring for 20 minutes the temperature was raised to 20°C and solid 3-ethoxy-14-alpha-hydroxy-androsta-3,5-dien-17-one (10 g, 30.3 mmol) was added. The reaction mixture was stirred for 30 minutes, cooled in ice/water, and water (100 ml) was carefully added. The organic layer was separated and washed with water and diluted acid.

The aqueous layer was extracted with toluene. The organic layers were combined, dried and evaporated in vacuo. Hexane (200 ml) was carefully added to the residue. After cooling at 0°C for 30 minutes the crystallized mass was filtered off, washed with hexane and dried. Hydrolysis of the 3-ethoxygroup resulted in 14-alpha-17-alpha-dihydroxy-17-beta-1-(1-phenylthioethenyl)-androst-4-en-3-one. NMR ($CDCl_3$, $CD_3OD$) delta 0.98 (3H, s), 1.27 (3H, s), 4.20 (2H, s), 4.78 and 5.51 (2H, 2s), 5.87 (1H, s), 7.1 (5H, m).

## Example IX

## 14-alpha-17-alpha-Propylidenedioxy-pregn-4-ene-3,20-dione

The reaction product of Example VIII was dissolved in methylenechloride (60 ml). Acetic acid (60 ml), water (6 ml) and a solution of sulphuric acid in acetic acid (3 ml, 10% v/v) were added. After stirring for 3 hours sodium hydroxide solution (2 ml, 50%) was added. Methylenechloride was evaporated in vacuo, followed by addition of 250 ml of water to the residue. The crystallized mass was filtered off, and washed with acetone (2 x 10 ml). The compound had a NMR spectrum which was identical with the compound prepared in Example VII.

The crude reaction product was converted into 14-alpha-17-alpha-propylidenedioxy-pregn-4-ene-3,20-dione according to known methods. Yield: 4.53 g (39% calculated on 3-ethoxy-14-alpha-hydroxy-androsta-3,5-dien-17-one). The compound was identical with the compound prepared in Example VI.

## Example X

## 14-alpha-17-alpha-Dihydroxy-3-ethoxy-17-beta-1-(1,1-trimethylenedithioethyl)-androsta-3,5-diene

To a solution of 1,1-trimethylenedithio-ethane (20 ml, 2-methyl-1,3-dithiane) in dry THF (200 ml) under a nitrogen atmosphere n-butyllithium (100 ml, 15% in hexane) was added in 15 minutes at -50°C. After stirring for 1 hour at 0°C

solid 3-ethoxy-14-alpha-hydroxy-androsta-3,5-dien-17-one (15 g, 45.45 mmol) was added. The reaction mixture was stirred for 30 minutes at 0°C and water (200 ml) was added. The reaction mixture was extracted with toluene, the organic layer was washed with water, dried and evaporated in vacuo. Hexane (150 ml) was carefully added to the residue. The crystallized mass was filtered off after 10 hours, washed with hexane and dried. Hydrolysis of the 3-ethoxy group resulted in 14-alpha-17-alpha-dihydroxy-17-beta-1-(1,1-trimethylenedithioethyl)-androst-4-en-3-one. NMR ($CDCl_3$) delta 1.210 (6H, s), 2.0 (3H, s), 4.45 (2H, s), 5.70 (1H, s).

## Example XI

### 14-alpha-17-alpha-Dihydroxy-pregn-4-ene-3,20-dione

The title compound was prepared from the reaction product of Example X according to the method described in Example IX. The product was identical with the product prepared in Example VII. The isolated product was converted into 14-alpha-17-alpha-propylidenedioxy-pregn-4-ene-3,20-dione. Yield 9.45 g (54 % calculated on 3-ethoxy-14-alpha-hydroxy-androsta-3,5-dien-17-one). The product was identical with the product prepared in Example VI.

## Example XII

### 14-alpha-17-alpha-Dihydroxy-3-ethoxy-17-beta-1-(1-ethoxy-ethenyl)-androsta-3,5-diene

n-Butyllithium (200 ml, 15% in hexane) was concentrated by destillation of hexane (100 ml) under a nitrogen atmosphere. THF (250 ml) was added to the residue at 0°C. Ethyl vinylether was added to the THF in 10 minutes. The reaction mixture was refluxed for 20 minutes, cooled to 25°C and solid 3-ethoxy-14-alpha-hydroxy-androsta-3,5-dien-17-one (10 g, 30.3 mmol) was added. The reaction mixture was stirred for 30 minutes and water (100 ml) was added at 0°C. The

organic layer was separated and washed with water and ammonium chloride solution. The aqueous layer was extracted with toluene. The organic layers were combined, washed, dried and evaporated in vacuo. The title compound was obtained as a white, solid mass.

Example XIII

14-alpha-17-alpha-Dihydroxy-pregn-4-ene-3,20-dione

The title compound was prepared from the reaction mixture of Example XII according to the method described in Example IX. The isolated compound was converted into 14-alpha-17-alpha-propylidenedioxy-pregn-4-ene-3,20-dione. Yield: 7.49 g (64% calculated on 3-ethoxy-14-alpha-hydroxy-androsta-3,5-dien-17-one). The isolated compounds were identical with the products prepared in Example VII and VI respectively.

Example XIV

14-alpha-17-alpha-Dihydroxy-17-beta-1-(1-ethoxyethenyl)-androst-4-en-3-one

To a solution of ethyl vinylether (50 ml) in dry THF (50 ml) under a nitrogen atmosphere n-butyllithium (100 ml, 15% in hexane) was added. After refluxing for 20 minutes solid 14-alpha-hydroxy-3-(N-morfolino)-androsta-3,5-dien-17-one (5 g, 14.1 mmol) was added at 25°C. After stirring for 30 minutes water (50 ml) was carefully added. The reation mixture was separated and washed with water and sodium bicarbonate solution. The aqueous layer was extracted with toluene. The organic layers were combined and evaporated in vacuo. The resulting oil was added to a solution of sodium bicarbonate (42 g) in water (65 ml) and ethanol (175 ml). After refluxing under a nitrogen atmosphere the solid was filtered off and washed with ethanol. The filtrate was concentrated, and water was carefully added. The crystallized compound was filtered off, washed with ethanol/water and filtered off. Yield 4.12 g (78%) of the title compound. NMR ($CDCl_3$) delta 0.803 (3H, s), 1.200 (3H, s), 1.32 (3H, tr), 3.64 (1H, s), 3.73 (2H, q), 4.07 and 4.30 (2H, 2 x d), 4.64 (1H, s), 5.73 (1H, s).

Example XV

14-alpha-17-alpha-Dihydroxy-17-beta-1-(1-ethylsulfenyl-ethenyl)-androst-4-en-3-one.

To a solution of ethyl vinylsulfide (0.47 g, 5.34 mmol) in dry THF (20 ml) under a nitrogen atmosphere t-butyllithium (1.7 M in pentane, 3 ml) was carefully added at -70°C. After stirring for 1,5 hours at -75°C the temperature was raised to 0°C and solid 3-ethoxy-14-alpha-hydroxy-androsta-3,5-dien-17-one (0.33 g, 1 mmol) was added. The reaction mixture was stirred for 45 minutes at 0°C, after which water (1 ml) and concentrated aqueous hydrogen chloride solution (1 ml) was added. After stirring for 20 minutes the reaction mixture was neutralized with a bicarbonate solution and was extracted with ethyl acetate, the organic layer washed with a sodium chloride solution, filtered, dried and evaporated in vacuo. Yield 164 mg (42%) of the title compound. $^1H$ NMR ($CDCl_3$) delta 0.849 (3H, s, 1.207 (3H, s), 1.32 (3H, tr), 4.17 and 4.34 (2H, 2 x s), 4.95 and 5.45 (2H, 2 x s), 5.72 (1H, s).

The compound could be converted into 14-alpha-17-alpha-dihydroxy pregna-4-ene-3,20-dione. Therefore, experiment was repeated under the same conditions, except that the reaction mixture was hydrolized for 1,5 hours. Yield 66% (calculated on 3-ethoxy-14-alpha-hydroxy-androsta-3,5-dien-17-one. The NMR spectrum was identical with the product obtained in Example VII.

Example XVI

14-alpha-17-alpha-Dihydroxy-17-beta-1-(1-phenylsulfinyl-ethenyl)androst-4-en-3-one

To a solution of diisopropylamine (1 ml, 7.08 mmol) in dry THF under a nitrogen atmosphere n-butyllithium in hexane (5.5 ml, 15%) was added in 10 minutes at -25°C. After stirring for 5 minutes a solution of phenylsulfinylethene (1.2

g, 7.89 mmol) in THF (4 ml) was added at -70°C. The reaction mixture was stirred for 15 minutes at -75°C. The temperature was raised to -10°C and solid 3-ethoxy-14-alpha-hydroxy-androsta-3,5-dien-17-one (0.5 g, 1.52 mmol) was added. The reaction mixture was stirred for 30 minutes at -20°C. After which water (5 ml) and concentrated aqueous hydrogen chloride solution (4 ml) were added. After stirring for 30 minutes at 0°C the reaction mixture was extracted with ethyl acetate. The organic layer was washed with water and 1 M sodium bicarbonate solution, dried and evaporated in vacuo. Chromatography (silica gel, toluene/aceton 5/1) afforded the two isomers of the title compound (I: 220 mg, II: 140 mg)

ISOMER I: NMR ($CDCl_3$, $CD_3OD$) delta 0.88 (3H, s), 1.19 (3H, s), 4.46 (2H, s), 5.68 (2H, s), 6.07 and 6.37 (2H, 2 x d), 7.4-7.8 (5H, m).

ISOMER II: NMR ($CDCl_3$, $CD_3OD$) delta 0.92 (3H, s), 1.26 (3H, s), 4.43 (2H, s), 5.71 (2H, s), 5.92 and 6.32 (2H, 2 x d), 7.3-7.8 (5H, m).

## Example XVII

**14-alpha-17-alpha-Propylidenedioxy-17-beta-1-(1-phenylsulfinylethenyl)-androst-4-en-3-one.**

A solution of 14-alpha-17-alpha-dihydroxy-17-beta-1-(1-phenylsulfinylethenyl)-androst-4-en-3-one (isomer I of Example XVI, 100 mg, 0,22 mmol) in acetic acid/propanal/-concentrated sulphuric acid (1 ml, 25/4/0.15 v/v/v) was stirred for 1.5 hour after which the reaction mixture was poured into a sodium bicarbonate solution and extracted with methylene chloride. The organic layer was washed with water and evaporated in vacuo. After crystallization from acetone/-hexane the title compound was obtained (yield 60 mg). NMR ($CDCl_3$) delta 0.92 (3H, tr), 0.924 (3H, s) 1.150 (3H, s), 4.75 (1H, tr), 5.73 (1H, s), 6.02 and 6.50 (2H, 2 x d), 7.4-7.9 (5H, m).

The other isomer was prepared according to the above starting from isomer II of Example XVI (Yield 71 mg). NMR ($CDCl_3$) delta 0.71 (3H, tr), 0.920 (3H, s), 1.200 (3H, s), 4.64 (1H, tr), 5.73 (1H, s), 5.87 and 6.48 (2H, 2 x d), 7.3-7.8 (5H, m).

## Claims

1. Process for the preparation of 14-alpha-17-alpha-dihydroxy-17-beta-substituted steroids, characterized in that a 14-alpha-hydroxy-17-oxo-steroid is reacted with a metal organic compound.

2. Process according to claim 1, characterized in that the metal organic compound has the formula R-M in which R represents a straight or branched alkyl, 1-alkenyl or 1-alkynyl group which optionally may contain a double bond and which optionally may be substituted by one or more substituents and M represents a lithium, sodium or potassium atom.

3. Process according to claim 2, characterized in that R represents a straight or branched $C_{1-6}$ alkylgroup, preferably methyl or ethyl, a $C_{1-6}$ 1-alkenylgroup, preferably ethenyl, or a $C_{1-6}$ 1-alkynylgroup, preferably ethynyl, which groups may be substituted by one or more $C_{1-4}$ alkoxy, aryloxy, tetrahydropyranyloxy, $C_{1-4}$ alkylthio, arylthio, $C_{1-4}$ alkylsulfinyl, arylsulfinyl, $C_{1-4}$ alkylsulfonyl, arylsulfonyl, $C_{1-4}$ dialkylamino, $C_{1-4}$ alkyl-arylamino, diarylamino, $C_{1-6}$ alkylhydrazono, $C_{1-4}$ alkylenedioxy, $C_{1-4}$ alkyleneoxythio, $C_{1-4}$ alkylenethiosulfoxy or $C_{1-4}$ alkylenedithio groups or a 1-(tri-$C_{1-4}$ alkylsilyloxy) or di $C_{1-6}$ alkylamino group together with a 1-cyano group, the aryl groups preferably phenyl or napthyl, optionally substituted by a methyl group.

4. Process according to claim 2, characterized in that R represents an ethyl group, an ethenyl group or an ethynyl group, which groups may be substituted by one or more $C_{1-4}$ alkoxy, phenyloxy, $C_{1-4}$ alkylthio, phenylthio, $C_{1-4}$ alkylsulfinyl, phenylsulfinyl, $C_{1-4}$ alkylsulfonyl, phenylsulfonyl, $C_{1-4}$ dialkylamino, $C_{1-4}$ alkyl-phenylamino, diphenylamino, $C_{1-4}$ alkyleneoxythio, $C_{1-4}$ alkylenethiosulfoxy or $C_{1-4}$ alkylenedithio groups, the phenyl groups optionally substituted by a methyl group, or by a 1-tri-($C_{1-4}$ alkylsilyloxy) group together with a 1-cyano group.

5. Process according to claim 4, characterized in that R represents an ethyl group substituted at the 1 position by two groups choosen from $C_{1-4}$ alkoxy, phenyloxy, $C_{1-4}$ alkylthio, phenylthio, $C_{1-4}$ alkylsulfinyl, phenylsulfinyl, $C_{1-4}$ alkylsulfonyl and phenylsulfonyl, or a 1-cyano group together with a tri-($C_{1-4}$ alkylsilyloxy) group, or substituted at the 1 position with a $C_{1-4}$ alkyleneoxythio, $C_{1-4}$ alkylenethiosulfoxy or $C_{1-4}$ alkylenedithio group, the phenyl groups optionally substituted by a methyl group.

6. Process according to claim 4, characterized in that R represents an ethenyl group substituted at the 1-position with a group choosen from $C_{1-4}$ alkoxy, phenyloxy, $C_{1-4}$ alkylthio, phenylthio, $C_{1-4}$ alkylsulfinyl, phenylsulfinyl, $C_{1-4}$ alkylsulfonyl and phenylsulfonyl, the phenyl groups optionally substituted by a methyl group.

7. Process according to claim 4, characterized in that R represents an ethynyl group.

8. Process according to claim 2, characterized in that the metal organic compound is lithium acetylide, preferably in combination with diaminoethane, 1-lithio-1,1-trimethylenedithio-ethane, 1-lithio-1-ethylthio-ethene, 1-lithio-1-phenylthio-ethene, 1-lithio-1-phenylsulfinylethene, 1-lithio-1-phenylsulfonyl-ethene, 1-lithio-1-ethoxy-ethene or 1-lithio-1-phenoxy-ethene.

9. Process according claims 1-8, characterized in that the 14-alpha-hydroxy-17-oxo-steroid has the general formula

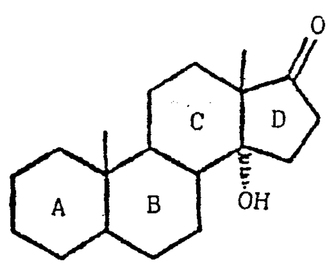

III

in which the rings A, B, C and D optionally contain one or more double bonds, are substituted or not substituted by one or more hydroxy groups, amino groups, oxygen atoms or alkoxy or alkoxyalkoxy groups and are disubstituted or not disubstituted by one or more alkylenedioxy, alkylenedithio, alkylenethiosulfoxy or alkyleneoxythio groups.

10. Process according to claim 9 in which the rings A, B, C and D contain one or more double bonds between $C_1$ and $C_2$, $C_3$ and $C_4$, $C_4$ and $C_5$, $C_5$ and $C_6$, $C_6$ and $C_7$, $C_9$ and $C_{11}$ and/or $C_{11}$ and $C_{12}$.

11. Process according to claims 9-10, in which the rings A, B, C and D are substituted by one or more 3-, 9-, 11- or 12-hydroxy group, and/or by an oxygen atom at $C_3$, $C_{11}$ or $C_{12}$ and/or by a 3-, 9- or 11-alkoxy group containing 1-4 carbon atoms, and/or by a 3-alkoxyalkoxy group.

12. Process according claims 9-11, in which the rings A, B, C and D are disubstituted by a 3,3-alkylenedioxy, a 3,3-alkylenedithio, a 3,3-alkylenethiosulfoxy or a 3,3-alkyleneoxythio group.

13. Compounds of the general formula IV

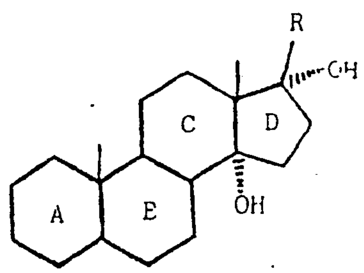

IV

wherein R is defined in claims 2-8 and the substituents of the steroid molecule are as defined in claims 9-12.

14. Process for the preparation of compounds of the general formula V

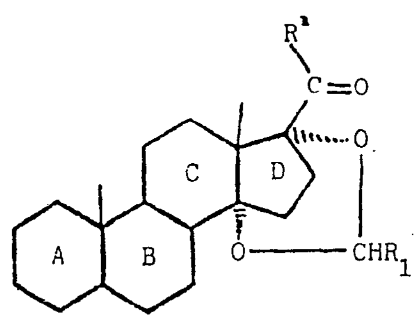

V

starting with a compound of the general formula IV, wherein R' represents the remaining part of the group R after hydrolysis into the 17-beta-acyl group whereby R is defined in claims 2-8, and the substituents of the steroid molecule are as defined in claims 9-12 and $R_1$ represents a straight or branched chain aliphatic hydrocarbon group having less than 10 carbon atoms which is optionally substituted by a halogen atom or by a carboxyl or methoxycarbonyl group, an aryl group which is optionally substituted by a halogen atom, or by a $C_{1-6}$ alkyl, nitro or amino group or by one or more lower alkoxy groups or an aryl ($C_{1-6}$ alkyl) group, a $C_3$-$C_8$ cycloalkyl group, a lower alkoxy group or the furyl group, characterized in that a compound of the formula IV as described in claim 13 is reacted with an aldehyde of the general formula $R_1CHO$ or an acetal thereof or a suitable othoformate, followed by conversion of the group R into an acyl group, or that the group R is converted into an acyl group followed by reaction with the aldehyde of the general formula $R_1CHO$ or an acetal thereof a suitable orthoformate.

110185/15

European Patent Office

0188010

Application number

# EUROPEAN SEARCH REPORT

EP 85 20 0022

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| A | EP-A-0 053 845 (MITSUBISHI)<br>* Claims 1-6 *<br>--- | 1 | C 07 J 7/00<br>C 07 J 31/00<br>C 07 J 71/00 |
| A | GB-A-1 218 282 (KONIKLIJKE NEDERLANDSCHE GIST- & SPIRITUSFABRIEK)<br>--- | | |
| A,D | FR-A-2 017 798 (KONINKLIJKE NEDERLANDSCHE GIST- & SPIRITUSFABRIEK)<br>----- | | |
| | | | TECHNICAL FIELDS SEARCHED (Int. Cl.4) |
| | | | C 07 J 7/00<br>C 07 J 1/00 |

The present search report has been drawn up for all claims

| Place of search | Date of completion of the search | Examiner |
|---|---|---|
| THE HAGUE | 16-09-1985 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82